# EUROPEAN PATENT SPECIFICATION

(11) **EP 3 939 551 B1**
(45) Date of publication and mention of the grant of the patent: **29.11.2023**
(21) Application number: 19722657.4
(22) Date of filing: 12.03.2019
(51) Int. Cl.: A61F 5/01, A61F 5/02

(54) **LUMBAR PROTECTION EXOMUSCLE**
LWS-SCHUTZ-EXOMUSKEL
EXOMUSCLE DE PROTECTION LOMBAIRE

(43) Date of publication of application: 19.01.2022
(73) Proprietor: Cainzos Pérez, Victor José, 08903 L'Hospitalet de Llobregat (ES)
(72) Inventor: Cainzos Pérez, Victor José, 08903 L'Hospitalet de Llobregat (ES)
(74) Representative: Espiell Gomez, Ignacio
(86) International application number: PCT/ES2019/070168
(87) International publication number: WO 2020/183037

(56) References cited:
- EP-A2- 1 293 188
- DE-A1-102017 123 518
- US-A1- 2005 130 815
- US-B1- 6 190 342
- US-B1- 6 436 065

## Description

The invention disclosed in the current specification refers to an exomuscle for lumbar protection. It reduces significantly and mainly the force exerted by the lumbar and other muscle groups such as spinal erectors, gluteus maximus and femoral biceps. It achieves so by using a harness which is fastened in different strategic areas such as the shoulders, hip, knees and ankles, being these fasteners connected to each other at the rear part of the body by textile materials, thus creating a vertical structure that traverses the entire rear part of the body (from shoulders to feet), and which is held tight at all times. In addition, these supports effectively distribute the forces generated by the lumbar exomuscle.

The functionality of the system lies in that, between the shoulders and the hip, and the knees and the ankles, the materials are not elastic, whereas between the hip and the knees, an elastic material with vertical elasticity is positioned preferably with a height regulator, being this area the only one that is stretched and deformed at the moment of flexion of the hip. Thus it generates a physical resistance that simulates the muscular function of the muscles responsible for lumbar damping, support and extension.

### TECHNICAL FIELD

The lumbar exomuscle proposed must be regarded as an orthosis, since the term exoskeleton does not conform to its reality due to the absence of rigid pieces simulating bone structures. Moreover, the purpose of the device is to be an assistive technology oriented to occupational therapy. Assistive technologies comprehend any product (including devices, equipment, tools, technology and software) that corrects or facilitates the execution of an action, activity or movement, seeking to save energy and achieve greater safety by improving the function of the locomotor system.

### PRIOR ART

As it is known, the spinal column is constituted by a chain of vertebrae linked to each other by intervertebral discs, connected to the pelvis, and this, to the bone system of the lower limbs. This whole structure is assembled by the paravertebral muscles, abdominal muscles, quadratus lumborum, psoas, glutes and pyramidal and femoral biceps, allowing the flexion and extension movement of the whole body.

Continuous movements and efforts of the muscles responsible for the movement of the involved joints influence the health of the anatomy of the lumbar region, such as weight loads, incorrect habits, flexed posture in a continuous way, working conditions, etc.

All these agents influence decisively the appearance of injuries in the musculoskeletal system such as osteoporosis of the vertebrae, muscular contracture, disc rupture or herniation, degeneration of intervertebral discs, misalignment of vertebrae, spinal stenosis, muscle or ligament strain or tear, abnormal spine curvatures, etc. All of these pathologies will cause pain and discomfort that can become chronic and severely affect the activities of the daily life of the user.

In other cases, some genetic factors such as anatomical defects of the spinal column have pathological nature, as in the case of kyphosis, lordosis or scoliosis, said alterations being an added risk for the development of the aforementioned pathologies.

To date, the main assistive technologies used to attempt to reduce the overload of the lumbar area at the bending moments are lumbar braces, since the pressure they exert on the contour of the abdomen limits the movement and the muscular function of the lumbar area. The problem is that this pressure has many drawbacks for the health of the user. In addition, it should be stressed that lumbar braces have not been conceived nor are recommended for said use.

Currently, mechanical artifacts called exoskeletons have been developed, whose function might be the same as that of the lumbar exomuscle, but unlike it, such exoskeletons use rigid structures, machined or not, that beyond their function, cause great drawbacks because they exert pressure over the soft tissues, given the need for support of those rigid structures and the forces they generate on the skeleton of the user, compressing and damaging the soft tissues.

Patent documents EP1293188, US2005/130815 and US6190342 are examples of exomuscles using elasting and non-elastic elements.

Patent document US6436065 discloses a lumbar protection exomuscle according to the preamble of claim 1. The back piece is rigid and cranked, with a lower part designed to fit the user at the hight of the hips.

After these observations, we can state that the claimed lumbar exomuscle performs, in a more efficient and effective manner, the function of the aforementioned products. Aiding the musculoskeletal system at the bending moments, thanks to not limiting muscle mobility as lumbar braces do, but accompanying the muscle in its movement. Moreover, the exomuscle is much lighter, ergonomic and healthy than an exoskeleton, for the simple fact that it is not a rigid structure that uses the body of the user as a lever point.

### DISCLOSURE OF THE INVENTION

Because of everything mentioned in the previous section, the demonstrated aid and reduction in the muscle effort by the claimed lumbar exomuscle generate large benefits in preventive aspects of possible musculoskeletal injuries to the lumbar area and, in addition, a significant reduction in the health and social care costs that this type of pathologies generate for the State and the companies themselves.

The invention corresponds to an orthosis, which we consider as an external muscle (exomuscle) in the form of a harness, and which is claimed in claims 1-4 and is described below, together with each of the parts thereof and the relationship between them.

Starting from the rear part of the waist upwards, passing through the rear part of the back, non-elastic fasteners are extended around the shoulders to create the upper support and fastening.

Over the waist area, a support is fastened horizontally around it, thus creating another support point that not only distributes the forces generated, but also prevents lateral displacements of the lumbar exomuscle.

From the support fastened at the height of the hips, elastic deformable materials are extended through the rear part over the gluteus and femoral biceps in vertical direction to the knee fastener. The materials in this area have the function of deforming at the moment when a flexion of the hip occurs, thus creating an elastic tension that simulates the directional force exerted by the muscles of the musculoskeletal system of the aforementioned area.

For securing the elastic deformable materials of the lower end, fasteners are placed horizontally around the knees, specifically on the lower part of the patella. The function of these fasteners is to provide a good anchoring point when the elastic deformable materials generate vertical forces and to maintain them on the rear part of the gluteus and femoral biceps.

Finally, from the knee's fasteners, non-elastic fasteners will extend to the feet area, fastening to them. The function of said fasteners is to subtract tension from the knee's fasteners and to maintain them in a much more comfortable position.

It has also been foreseen that such fasteners may not fit all anatomies, therefore the invention has been provided with the possibility of regulating them to place them at the height and width most suitable in each case. In this regard, it has been arranged that, together with the fasteners of the shoulders, knees and heels, there are adjusting elements that can be regulated both in height and width.

This way, a current problem is solved: a great number of people suffer, or have suffered at some point in their lives, from ailments in the lumbar area caused by an overexertion in said area, since the aforementioned lumbar exomuscle exerts a force aimed to absorb this overexertion of the user's lumbar area, with the consequent decrease of the possibility of injury and palliation of the ailments already existing.

The lumbar protection exomuscle comprises a harness formed by an back piece, two elastic straps and two foot-fasteners. The back piece comprises shoulder straps and a hip strap. The two elastic straps are affixed to the bottom part of the back piece. Each elastic strap comprises a knee fastener for wrapping around the lower area of the patella, or any other relevant area of the user. A foot fastener extends from each elastic strap, stretching to the foot and clasping to the contour of the foot or ankle.

The harness might comprise several regulators that allow for the adaptation in size of each element, mainly the straps or fasteners.

In a preferred embodiment, the foot fastener comprises an element of footwear: shoe or sock.

Elastic material will be understood as any material which can return to its original shape and size after the expected deformation during the use of the exomuscle. It may also comprise a combination of non-elastic and elastic elements.

### DESCRIPTION OF THE DRAWINGS

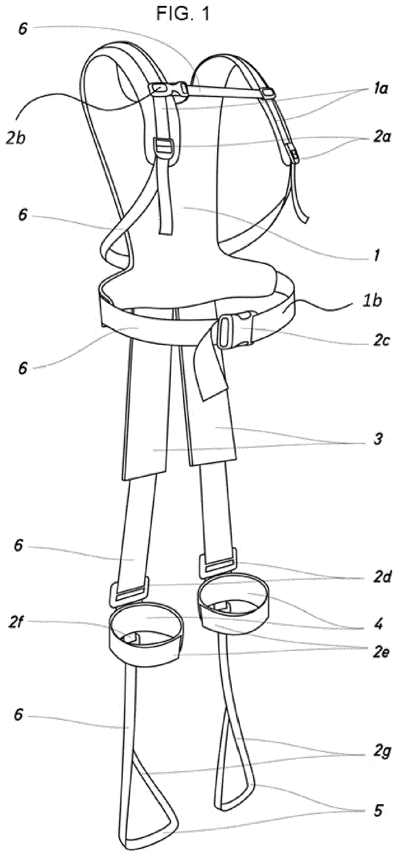

To complete the description being made and in order to aid a better understanding of the characteristics of the invention, according to an example of a preferred embodiment thereof, as an integral part of the description, a drawing (fig. 1) is provided for the purposes of illustration only and in a non-limiting manner.

### PREFERRED EMBODIMENT OF THE INVENTION

The lumbar exomuscle of the invention relates to a harness that comprises elastic textile materials, or of any other type, in its main parts with the exception of the anchors and regulators, which may be of any hard material such as plastic or metal. A back piece (1) is placed at the back and the shoulder straps (1a) that integrate it are intended for the adaptation of the device to the user around the shoulders and hip. The shoulder straps (1a) are not elastic so as to not to detract from the effectivity of the device. The piece (1) is equipped with several regulators (2) that allow for the adaptation in height size with the regulators of the shoulder straps (2a), in chest contour and height with the chest regulator (2b) and in waist contour with the hip regulator (2c) of a hip strap (1b).

Linked to the back piece (1), two elastic straps (3) are joined at the bottom part, either textile or of any other elastic material that accumulates energy in the moment of flexion. The two elastic straps (3) are adjustable in height thanks to the lower regulator (2d) located at their lowest part. To withstand the force generated by the elastic straps (3), knee fasteners (4) are arranged wrapping around the lower area of the patella, or any other relevant area of the user. The knee fasteners (4) are made of textile materials or any other type of material, which allow for comfortable and effective adaptation thereof to the contour of the knees and keep them in place using some type of contour regulator (2e).

In order to avoid the overload caused by the knee fasteners (4), extending therefrom, there are foot fasteners (5) stretching to the foot and clasping to its contour or any other suitable area. Such foot fasteners (5) are made of textile materials or any other type of material whose deformation is minimal so as to not to detract from the effectivity of the device. In addition, the knee fasteners (4) also are adjustable by a height regulator (2f) located in the upper part and by another width regulator (2g) placed around the feet. Finally, each regulator (2) has a strap (6) of textile material or of any other non-elastic type, that allows for a margin of adjustment of the device without causing it to lose effectivity. That is, the strap (6) provides the material for the enlengthening or stores the material when shortening the corresponding part of the exomuscle.

## Claims

1. Lumbar protection exomuscle that comprises a harness formed by:
• a rigid back piece (1) with shoulder straps (1a) and a hip strap (1b) such that the back piece (1) is adapted to the user around the shoulders and hip when the lumbar protection is worn by the user;
• two elastic straps (3) affixed to the bottom part of the back piece (1);
• two knee fasteners (4) for wrapping around the lower area of the patella;
• two non-elastic straps (6) between the two elastic straps (3) and the two knee fasteners (4); and
• two foot fasteners (5) extending from each non-elastic straps (6) stretching to the foot and clasping to the contour of the foot or ankle,
**characterized in that** the hip strap is arranged in correspondence to the bottom part of the rigid back piece and **in that**
an upper part of the elastic straps (3) affixed to the bottom part of the back piece (1) is located over the hips of the user when the lumbar protection is worn by the user.

2. Lumbar protection exomuscle, according to claim 1, that further comprises several regulators (2) that allow for the adaptation in size.

3. Lumbar protection exomuscle, according to claim 1, where the foot fastener comprises an element of footwear.

4. Lumbar protection exomuscle, according to claim 1, where the back piece (1) comprises non-elastic fasteners from the rear part of the waist upwards, passing through the rear part of the back, and extended around the shoulders to create the upper support and fastening.

## Patentansprüche

1. Exomuskel zum Schutz der Lendenwirbelsäule, der einen Gurt umfasst, der gebildet wird durch:
• ein starres Rückenstück (1) mit Schulterbändern (1a) und einem Hüftband (1b), sodass das Rückenteil (1) um die Schultern und die Hüfte an den Benutzer angepasst ist, wenn der Lendenwirbelschutz vom Benutzer getragen wird;
• zwei elastische Bänder (3), die am unteren Teil des Rückenstücks (1) befestigt sind;
• zwei Kniebefestigungen (4) zum Umwickeln des unteren Bereichs der Kniescheibe;
• zwei nicht-elastische Bänder (6) zwischen den beiden elastischen Bändern (3) und den beiden Kniebefestigungen (4); und
• zwei Fußbefestigungen (5), die sich von jedem nicht-elastischen Band (6) aus erstrecken, das sich um den Fuß spannt und sich an die Kontur des Fußes oder Knöchels anschmiegt,
**dadurch gekennzeichnet dass** das Hüftband in Übereinstimmung mit dem unteren Teil des starren Rückenstücks angeordnet ist und dass ein oberer Teil der elastischen Bänder (3), die am unteren Teil des Rückenstücks (1) befestigt sind, sich über den Hüften des Benutzers befindet, wenn der Lendenwirbelschutz vom Benutzer getragen wird.

2. Exomuskel zum Schutz der Lendenwirbelsäule nach Anspruch 1, der ferner mehrere Regulatoren (2) umfasst, die die Anpassung der Größe ermöglichen.

3. Exomuskel zum Schutz der Lendenwirbelsäule nach Anspruch 1, wobei die Fußbefestigung ein Element des Schuhwerks umfasst.

4. Exomuskel zum Schutz der Lendenwirbelsäule nach Anspruch 1, wobei das Rückenstück (1) nicht-elastische Verschlüsse vom hinteren Teil der Hüfte nach oben umfasst, die durch den hinteren Teil des Rückens verlaufen und sich um die Schultern herum erstrecken, um die obere Stütze und Befestigung zu bilden.

## Revendications

1. Exomuscle de protection lombaire qui comprend un harnais formé par :
• une pièce de dos rigide (1) avec des sangles d'épaule (1a) et une sangle de hanche (1b) de telle sorte que la pièce de dos (1) est adaptée à l'utilisateur autour des épaules et de la hanche lorsque la protection lombaire est portée par l'utilisateur ;
• deux sangles élastiques (3) attachées à la partie inférieure de la pièce de dos (1) ;
• deux fixations de genou (4) pour envelopper la zone inférieure de la rotule ;
• deux sangles non élastiques (6) entre les deux sangles élastiques (3) et les deux fixations de genou (4) ; et
• deux fixations de pied (5) s'étendant depuis chacune des sangles non élastiques (6) s'étirant jusqu'au pied et épousant le contour du pied ou de la cheville,
**caractérisé en ce que** la sangle de hanche est agencée en correspondance avec la partie inférieure de la pièce de dos rigide et **en ce qu'**une partie supérieure des sangles élastiques (3) attachées à la partie inférieure de la pièce de dos (1) est située sur les hanches de l'utilisateur lorsque la protection lombaire est portée par l'utilisateur.

2. Exomuscle de protection lombaire, selon la revendication 1, qui comprend en outre plusieurs régulateurs (2) qui permettent l'adaptation de taille.

3. Exomuscle de protection lombaire, selon la revendication 1, où la fixation du pied comprend un élément de chaussure.

4. Exomuscle de protection lombaire, selon la revendication 1, où la pièce de dos (1) comprend des fixations non élastiques depuis la partie arrière de la taille vers le haut, passant par la partie arrière du dos, et étendues autour des épaules pour créer le support supérieur et la fixation.
